(19) Europäisches Patentamt
European Patent Office
Office européen des brevets

(11) **EP 1 700 595 B1**

(12) **EUROPEAN PATENT SPECIFICATION**

(45) Date of publication and mention
of the grant of the patent:
**25.02.2009 Bulletin 2009/09**

(51) Int Cl.:
***A61K 9/20*** *(2006.01)*    ***A61M 5/155*** *(2006.01)*
***A01N 25/18*** *(2006.01)*

(21) Application number: **04805104.9**

(22) Date of filing: **17.12.2004**

(86) International application number:
**PCT/ES2004/000561**

(87) International publication number:
**WO 2005/058285 (30.06.2005 Gazette 2005/26)**

(54) **TABLETS HAVING AN EMULSIFIED POLYMER MATRIX FOR THE CONTROLLED EMISSION OF GAS, AND PRODUCTION PROCESS**

TABLETTEN MIT EINER EMULGIERTEN POLYMERMATRIX ZUR KONTROLLIERTEN FREIGABE VON GAS UND HERSTELLUNGSVERFAHREN

TABLETTES AVEC MATRICE POLYMERE EMULSIONNEE POUR EMISSION CONTROLEE DE GAZ, ET PROCEDE DE FABRICATION

(84) Designated Contracting States:
**AT BE BG CH CY CZ DE DK EE ES FI FR GB GR HU IE IS IT LI LT LU MC NL PL PT RO SE SI SK TR**

(30) Priority: **18.12.2003 ES 200302997**

(43) Date of publication of application:
**13.09.2006 Bulletin 2006/37**

(73) Proprietor: **GAT Microencapsulation AG
2490 Ebenfurth (AT)**

(72) Inventors:
• **CASANA GINER, Victor
A-Ebenfurth 2490 (AT)**

• **GIMENO SIERRA, Miguel
A-Ebenfurth 2490 (AT)**
• **GIMENO SIERRA, Bárbara
A-Ebenfurth 2490 (AT)**
• **CONSTANTINE, Stephane
A-Ebenfurth 2490 (AT)**

(74) Representative: **Soler Lerma, Santiago et al
C/ Poeta Querol No. 1-10a
46002 Valencia (ES)**

(56) References cited:
**EP-A1- 0 976 395          WO-A1-00/23045
CN-A- 1 283 388          ES-T3- 2 134 450
JP-A- 8 048 622          JP-A- 2000 051 340**

EP 1 700 595 B1

## Description

## Object of the Invention

The invention relates to tablets capable of producing gases in a controlled manner for their preferred use in apparatuses intended for the administration of medicaments, blood and derivatives, or serums, such that the gas exerts the primary control of the rate of release of the serum or medicament to the patient. The tablets are found in a matrix which allows the reaction between an acid and a base (or an enzyme substrate and an enzyme), appropriately chosen, to take place in a controlled manner, particularly with a constant gas evolution rate during the useful life of use of the tablet.

The invention relates to medical devices for administering substances to the patient as well as to techniques for the controlled emission of gases for their use at home or as fumigants.

## State of the Art

It is necessary for hospital purposes to obtain a method of continuous release of medicaments or serum or the like for their continuous (normally intravenous) injection in the patient. Devices allowing such use are known, but they usually require additional devices (for example metallic structures) that allow the fall by gravity of the solution to be injected. A way of improving and reducing the cost and number of devices necessary for a continuous injection of medicaments is to eliminate the need to place the drip above the injection site. This allows to be able to administer the necessary medicaments, blood, etc. to the patient in extreme situations (e.g. in car accidents) where it is not feasible to place the drip, which operates by gravity, above the patient. The use for the same purpose of peristaltic pumps which rely on a generally electric power supply is also known.

The technique of exerting pressure by means of the presence of a gas in a receptacle wherein the solution to be injected is located is known. As a non-limiting example, the solution to be injected may be found in a flexible (plastic) bag which is compressed with the presence of the gas or, according to another non-limiting example, the solution is found in a infusion device with a plunger, which is displaced by the presence of the gas, thus imitating a manual injection with a needle/plunger.

Many of these devices which use the presence of a gas as a way of controlling the injection rate of solutions to the patients are based on electrolytic methods where the presence of an electric current causes a reaction in which the gas is produced and carried to a receptacle wherein the solution to be injected is packaged in a plastic film. These devices may be electronically adjusted by regulating the voltage so as to produce a greater or smaller gas evolution. The great drawback of these devices is their high cost and the reliance on a electricity source, and in the particular case of running on batteries, the exhaustion thereof causes a sudden stop of the injection process both in the electrolytic mechanisms and peristaltic pumps, apart from a high cost of the building materials of said drips.

The use of tablets consisting of sodium bicarbonate or sodium carbonate, which upon being combined with an acid -at the time the medical personnel wants to start the injection- (for example by rupture of a plastic film separating the acid from the basic tablet -the reaction thus starting-) cause the emission -by chemical reaction- of $CO_2$, is known. Other gas generating chemical reactions are possible (nitric oxide, nitrous oxide, hydrogen, etc.) but some are not convenient since either the generated gases are not adequate due to their toxicity or inflammability or the compounds that generate them are expensive.

On the other hand, traditional methods of disinfection or fumigation of soils intended for agricultural crops (also applicable e.g. to fumigation in industrial premises) make use of lethal gases which require great control in their handling. A typical example in the preplanting disinfection and fumigation of agricultural soils is the use of hydrogen cyanide or methyl bromide. The present invention shows a much safer method of applying these extremely toxic compounds thanks to the emission control of the tablets and their "low toxicity" form before their use (the reactive components are enclosed in the tablet, preferably with emetics, and provide a reduced toxicity by virtue of their enclosure in the tablet - the ingestion of which is prevented by emetics- before a solution causes the start of the toxic gas generating reactions).

All said tablets currently in the market as well as those described in patent (e.g. FR 27845832, EP 0669129, FR 2762213, WO 9811879) or scientific literature suffer from a pronounced gas evolution at the start of the chemical reaction between the acid and the base, the quantity of generated gas decreasing exponentially or at best linearly with a steep slope.

The present invention constitutes a marked improvement regarding the state of the art as far as the applicant has developed basic (or acid, or containing the acid and base in the same tablet) pills which upon reaction with an acid (or base, or tablet disintegrant) -or acid and basic at the same time- cause a constant gas evolution; in chemical terms it could be said the gas forming reaction is of an order of 0, and its emission goes on for a considerable time in comparison with the state of the art.

## Detailed Description of the Invention

Due to economic and toxicological reasons, the inventors have chosen as a preferred embodiment mode [only for a description of the invention in a practical manner] basic tablets (due to the presence of sodium carbonate) and a non-toxic acid such as citric acid. The following description avails itself of this non-liming example whatsoever such that the invention is understood in a practical and simple manner and is capable of being carried out by person skilled in the art.

The effervescent tablets are made such that the access of citric acid to sodium carbonate takes place in a controlled manner. Up until now, the only control described and known at least by the authors, the contact between acid and base, takes place without any type of physical or chemical protection, except for the use of films which separate the acid solution from the basic tablet. These described membranes are not capable of regulating the flow of acid inside the receptacle wherein the basic tablets are, or alternatively, the flow of alkali which comes in contact with acid tablets, or alternatively, the flow of water that dissolves the pills containing the acid and base, such that, because of a greater contact surface at the start of the reaction or.of a greater accessibility for the bonding of acid and base, a large gas evolution takes place at the beginning of the process. This gas evolution in commercial tablets studied by the inventors takes place in an uncontrolled manner, and in those described tablets (e.g. FR 27845832, EP 0669129, FR 2762213, WO 9811879) in which a hydrophilic polymer exerts the control of the reaction by means of channels that make way for water, these channels are rapidly destroyed by the "explosions" of water bubbles ($CO_2$). It is true that initially a control is indeed exerted by means of said inventions, but in no event whatsoever, according to the experiments carried out by the authors following the instructions present in said documents, may a regular control of the emission of $CO_2$ be achieved for more than 1 hour (for a tablet size and an emitted $CO_2$ quantity appropriate for exerting sufficient pressure for the actuation of the injection mechanism). It is to be noted that in none of said patents the use of these pills is described for causing $CO_2$ pressure; the use of those patents is intended for effervescence and is of permanence in the stomach. This time factor, then, is crucial thanks to the novel inclusion of a oil phase in this type of tablets, furthermore in the form of an emulsion integrated in a polymer matrix of a hydrophilic nature. In no event the combination of an emulsion with a hydrophilic polymer belongs to the state of the art. Furthermore, the existence of oil in the tablet no only exerts a fundamental effect on the diffusion of the liquid that the reaction produces (either alkaline, basic, neutral or even an enzyme solution, as will be seen below), but exerts a physical barrier against microexplosions of the formed gas, protecting the tablet structure (matrix) that controls the reaction. Moreover, the existence of the emulsion allows the inclusion of materials of a liquid nature in the tablets (e.g. a water-in-oil emulsion, the water containing enzymes that catalyze gas generating reactions).

The inventors present a way of controlling the physical contact of the base (or acid) with the acid (or base) by means of an innovative method of encapsulating the basic or acid tablet by means of an emulsion that regulates the diffusion of the acid or base or neutral compound in the tablet, this emulsion being of a polymeric nature. There are basically three combinations so that acid-base reaction takes place and three so that an enzymatic reaction occurs:

    Alkaline Tablet -------- Acid Solution
    Acid Tablet ------ Alkaline Solution
    Alkaline + Acid Tablet --------- Neutral Solution
    Enzymatic Tablet ----- Solution with Anabolite
    Tablet with Anabolite ----- Enzymatic Solution
    Tablet with Anabolite + Enzymes ----- Tablet Disintegrating/Solubilizing Solution

In a preferred embodiment mode of the invention, the effervescent tablets are made by means of the mixture of sodium carbonate in an emulsioned matrix of an oil in an ethylene glycol-type polymer. The emulsion is carried out with the aid of a dispersant (a polymer dispersant in a petroleum fraction with a high boiling point) and a sorbitan-type emulsifier, chosen in a non-limiting manner. Akofine R® (Karlshamms) and Atlox LP6® (Uniquema) are additives used in the present embodiment in an illustrative and non-limiting manner.

In a first step, the emulsion constituents are mixed and heated to an appropriate temperature that allows their fusion (preferably ranging from 50 to 100°C) in the presence of agitation with a polytron. Once the emulsion has an homogenous appearance, sodium carbonate is added to the emulsion. The sodium carbonate is granulated in very fine particles and must be perfectly homogenized regarding granule size. It is a factor of extreme importance since the size of the sodium carbonate granule will subsequently have a decisive influence on the production of $CO_2$ and its diffusion. The sodium carbonate is at the same temperature that the emulsion has, although it might be convenient to raise the temperature of the mixture of the emulsion (which consists of said hydroxylated polymers -which act as "aqueous" phase"- of a dispersant and the oil). Next, mixing, in an intense manner, of all of the components (sodium carbonate with the emulsion) is initiated such that obtaining a solid dispersion with the emulsion homogenously adsorbed in the sodium carbonate surface (or any other solid in other preferred embodiments) is ensured. Once the mixture is considered homogenized, the semisolid paste, to a greater or smaller degree of viscosity, is poured into molds which, after pressing and cooling, give raise to the tablets of sodium carbonate embedded in an emulsified polymer matrix. The composition by weight of the described tablet is sodium carbonate (48%)/ethylene glycol 4000 (25%)/ Akofine®(25%)/Atlox LP6® (1%)/Span 65(1%).

The inventors have discovered that in order to obtain this type of emulsion with great ease (not excluding other ways of carrying out the process), the fusion temperature of the components of the oil-hydrophilic polymer tablet must be similar, although this is not necessary.

The polymer matrix coats the solid sodium carbonate particles such that it takes a while for the citric acid to react with the sodium carbonate by virtue of the hydrophobic protective action of the emulsion or in particular of the oil, at the same time that there is a certain number of hydrophilic channels (non-emulsified polymer) protected against its destruction by the mechanical protective action of the oil.

Depending on the production conditions, the inventors have developed tablets that are capable of providing a constant emission of $CO_2$ for 30 minutes, 1 hour, 2 hours, 3 hours, 6 hours, 8 hours, 12 hours, 24 hours, 36 hours, and 48 hours. The differences used for prolonging the useful life of the tablet are based in the content of base (or acid in such embodiment), the degree of compaction of the tablet (increased with the life of the tablet), the quantity of hydrophilic polymer (reduced with the life of the tablet), the quantity of oil (increased with the life of the tablet), the existence of several layers of diverse degrees of compression and/or composition, additives, as well as other variations, particularly the pH of the solution that disintegrates the tablet. For example, the life of an alkaline tablet may be prolonged simply by reducing the acidity of the reacting acid solution (for example from pH=1 to pH=6).

The components necessary for producing $CO_2$ need not be citric acid nor sodium carbonate. Any pair of acid-base reagents which are capable of producing $CO_2$ are admissible in the described process and fall within the scope of the invention. Furthermore, any type of reaction that produces a gas is applicable, provided that the chemical foundations of the reaction do not differ excessively from those described herein (enzymatic reactions also included).

According to the investigation by the authors, the hydrophilic polymer may have a molecular mass of 100-500000 amus, preferably of 1000-3000 amus, and more preferably between 1000-8000 amus.

The oil phase may comprise natural or modified vegetable oils, (totally or partially, of course) hydrogenated vegetable oils, mineral oils, silicones, fluorinated silicones, modified silicones, or mixtures thereof in any proportion.

The tablets may contain additives:

(a) that prevent the deterioration thereof, preferably antimicrobial agents, antioxidants, UV blockers or filters.
(b) aromas that give off a characteristic smell, together with the evolution of gas, upon said tablets reacting with an acid or a base or upon disintegrating in contact with a solution.
(c) sequesterers of acids or bases.
(d) adsorbents/sequesterers of acids or bases of an organic (preferably EDTA) or inorganic (clays, sepiolites, zeolites) nature, including polymers (preferably based on citric or maleic acid remains integrated in the polymer, in the case of an alkaline tablet, preferably based on sulphonates, lignosulphonates, in the case of an acid tablet).

(e) salts that increase the ionic strength of the solution in which the tablet disintegrates.
(f) agglomerants or antiagglomerants, dispersants, stabilizers.
(g) emetics.

The hydrophilic-lipophilic balance of the emulsifier will significantly control the formation of a water-in-oil (HLB of 1-7) or oil-in-water (7-18) emulsion.

The acids (or their originating salts) are preferably chosen among the group: citric, lactic, phosphoric, benzoic, malic, maleic, malonic, fumaric, acetic, formic, propionic, succinic; as well as salts from these acids; these acids (or their mixtures) being emulsified-dispersed inside the tablet.

**Preferred Embodiment of the Invention**

In a preferred embodiment mode, a drip-type medical device is used wherein there is an aqueous solution of an acid, preferably 30-37% citric acid in water (w/w), separated by means of a sheet of plastic or plastic with aluminum from an alkaline tablet (preferably consisting of (a) partially hydrogenated vegetable oil (b) emulsified with a hydrophilic polymer, most preferably of a molecular mass between 2000-6000 amus, (c) an emulsifier of HLB 3-6, and (d) an alkali, preferably sodium carbonate); the sheet being broken by pressure right at the desired moment of using the drip; the $CO_2$ gas is then released by a controlled acid-base reaction upon the citric acid coming in contact with the bicarbonate and carbonate ions, and enters a chamber wherein the injectable liquid solution, which is encased in plastic, is found, preferably saline serum, serums with medicaments, blood or products derived from blood (encased in plastic), the $CO_2$ thereby creating pressure on the casing of the injectable solution which generates its flow towards the patient, this pressure being the primary regulator of the injection rate of the injectable solution.

In a preferred embodiment mode, the tablets are designed for the controlled emission of $O_2$, characterized in that they comprise a water-in-oil emulsion made up of:

(a) at least one emulsifier
(b) a solution of enzymes, preferably peroxidases
(c) at least one hydrophilic polymer
(d) hydrogen peroxide
(e) an oil phase
(f) optionally, additives;

said tablets being molded in a solid state at a temperature lower than 50 °C and applicable in controlled breathing systems both at a microbial and an animal or plant level, as well as in systems or processes requiring a continuous $O_2$ supply in a controlled manner (preferably chemical reactions, aquariums, chambers for microorganism growing, etc.), and the procedure for the controlled emis-

sion of $O_2$ is characterized in that the tablets formed according to Claim 21 come in contact with a solution containing enzymes of the peroxidase group, this solution produces $O_2$ upon the enzymes coming in contact with the hydrogen peroxide which is found in the tablet as a dispersed phase, together with the hydrophilic polymer, the oil being the continuous phase.

The tablets may also be used so that a gas aids in the volatilization of aromas for domestic use or for the enzymatic production of oxygen for the cleaning of toilets, bathrooms, etc.

A colorant or reaction indicator (either acid-base or enzymatic) may be added to the tablets in order to observe the appearance of color upon initiation of the reaction; optionally the intensity of the color may vary as the reaction develops.

It is understood that in the present invention the term "tablet" does not imply a geometric shape in particular.

## Brief Description of the Figures

**Fig. 1** shows the Emission of $CO_2$ of a sealed tablet (Tablet. 1) and a tablet exposed to humidity (Tablet. 2).

**Fig. 2** shows the influence of the oil phase concentration (w/w with regards to the hydrophilic phase) in the emission of $CO_2$.

**Fig. 3** shows the influence of the molecular weight of the propylene glycol polymer in the emission of $CO_2$.

**Fig. 4** shows the influence of the presence of salts (CINa 0,01 N) that increase the ionic strength of the emission rate of $CO_2$.

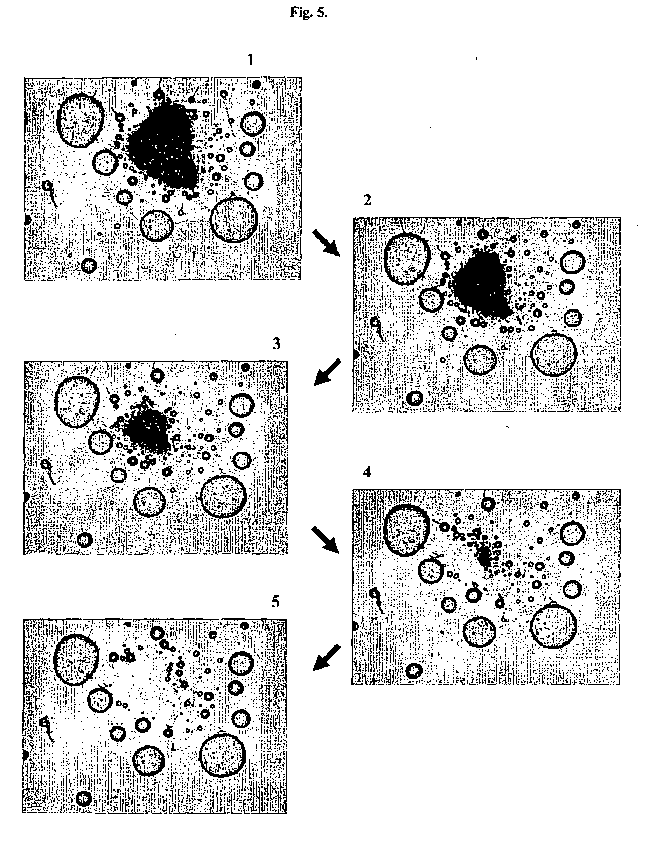

**Fig. 5** shows the disintegration without sudden rupture of the structure of the matrix of a basic tablet (containing carbonates of alkaline-earth metals -Ca, Mg-) in contact with an acid (lactic acid) in 5 steps for a 1 hour period. It is a microgranule of a tablet, which as a whole (the whole tablet) allows a controlled emission of $CO_2$ for 36 hours. The large-sized drops consist of drops of the water-in-oil emulsion, whereas the tiny drops correspond to the $CO_2$ released.

**Fig. 6** shows the microscopic structure of a tablet in accordance with the present invention, wherein white areas corresponding to the anhydrous sodium carbonate crystals, and the emulsified matrix consisting of a polypropylene glycol polymer of 3000 amus in a silicon oil can be seen.

**Fig. 7** shows a water-in-oil emulsion (before the mixture with the rest of matrix components) wherein a perfect enclosure in the borneol crystal emulsion as odorizing terpene for home application.

* The $CO_2$ evolution measurements have been carried out by gravimetric methods (decrease of the weight of a reactive solution in contact with the tablet).

## Examples

**Example 1.** Production of alkaline tablets for the emission of $CO_2$.

A procedure for making tablets containing sodium carbonate in order to produce -due to the contact with a citric acid solution- $CO_2$, this gas being used to compress the bag containing a medicament in a drip device adapted to this end, is shown in this example.

In a first step, an emulsion of the polymer Trans-P2000 K® and the hydrogenated vegetable glycerides Emuldan HV 40 Kosher® is formed. For this, they are mixed, heated to 80 °C, and at the moment a solution in a liquid-viscous state is obtained, the emulsifier (Span 65) is added. The emulsion is obtained by agitating with Ultraturrax® for 5 minutes (water-in-oil or oil-in-water, depending on the chosen emulsifiers and on the hydrophilic and oil phases, as well as on the proportion; in this case, an oil-in-water emulsion is obtained). The rheological parameters corresponding to this point are:

$$\eta \ (t = 1 \ Pa) \ = 0,268 \ Pa\text{*}s$$

$$\eta \ (t = 10 \ Pa) \ = 0,174 \ Pa\text{*}s$$

It is a water-in-oil emulsion.

At this moment, before the mixture with sodium carbonate, the formed emulsion has its limitations as regards stability. The applicant has discovered that the emulsion remains stable in the temperature range of 65-100 °C. At 150 °C the rupture of the emulsion starts to occur. Furthermore, the applicant has discovered that if the emulsion is slowly cooled and reheated, the rupture of the emulsion takes place with phase separation. However, if the emulsion is rapidly cooled, afterward, upon reheating, the emulsion remains stable.

In a following step, granulated anhydrous sodium carbonate [1:1, w/w] with a particle size smaller than 0.3 mm is mixed with the emulsion. This dispersion takes place under hot conditions (at 65-100 °C), the sodium carbonate being at 60°C -even though this is not necessary, it can help to prevent a thermal shock causing a phase inversion from happening-. Said mixture causes the adsorption coating of the sodium carbonate particles with emulsion films; also happening is that some sodium carbonate particles are not enclosed in the emulsion but are "free" although separated from one another by gaps where the emulsion is found. It is thus a heterogeneous mixture wherein the sodium carbonate is physically protected against aggressive (fast) acid attacks. In this regard, the inventors have discovered that when polymers that do not resist acids are used, the reaction of the acid with the sodium carbonate takes place in an uncontrolled manner (that is, not constant).

Finally, while the emulsion-sodium carbonate dispersion is still hot, the formed paste is molded in the shape of tablets thanks to molds of a flat, cylindrical shape arranged to such end (radius = 3 cm, height = 3 cm). The molds contain a multitude of cavities (with the desired tablet shape) wherein the paste (emulsion-dispersion) is introduced and slightly compacted by pressure.

Once the tablet cools down, it stays in a completely solid form, without any disintegration in the presence of air, light, or even at a relative humidity of 95%, for at least 5 days.

The tablet thus produced may be used in any utility model regarding medical devices so as to be the basic $CO_2$ source. If the tablet is mixed or dropped in a 36% citric acid solution [10 mL of citric acid per tablet gram] -acid form and acids chosen in an embodiment of the invention-, it will start to produce effervescence, that is, $CO_2$, and due to the innovative form developed after many selection tests of the type of oils and hydrophilic polymers and emulsification forms and concentrations, this $CO_2$ is released in a continuous manner and at a constant rate for 6 hours, improving the tablet-based, $CO_2$ releasing devices described to date.

**Example 2.** Influence of humidity on the emission rate of $CO_2$ of an alkaline tablet.

In the following example, the emission of $CO_2$ of a tablet produced in accordance with Example 1, with sodium carbonate as alkali, which has been prepared, packaged in a plastic vial, sealed with plastic-coated aluminum paper and kept at 19 °C, has been compared against that of a tablet which has been exposed to air with a humidity of 60% for 4 days at 19 °C.

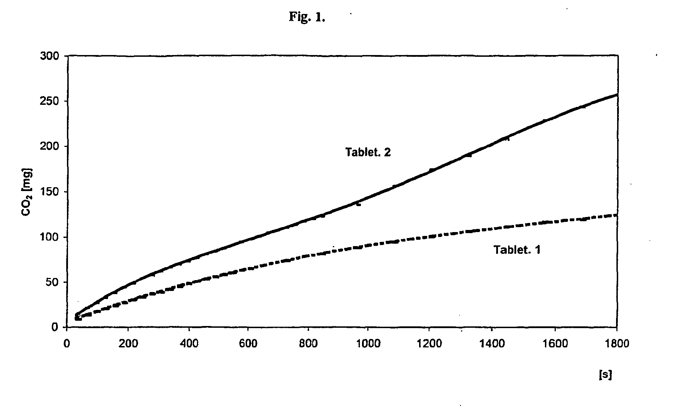

It can be seen in Fig. 1 how moistening the tablet before its use makes the emission of $CO_2$ greater than that of the reference tablet (without contact with atmospheric humidity), the emission rate in the first case being too high and not appropriate for a prolonged emission of $CO_2$ for more than 24 hours (by extrapolation).

**Example 3.** Influence of the quantity of oil phase on the emission rate of $CO_2$ of an alkaline tablet.

In the following example, a tablet produced with Praestol® as polymer, Promine® HV as emulsifier, and rosemary essential oil as oil phase is used to provide a pleasant smell at the site of emission of $CO_2$. The base

is made up of magnesium carbonate ($3MgCO_+$ Mg $(OH)_2{}^*3H_2O$), and the process has been carried out at 140 °C. The emission of $CO_2$ is compared depending on the percentage of rosemary essential oil in the tablet.

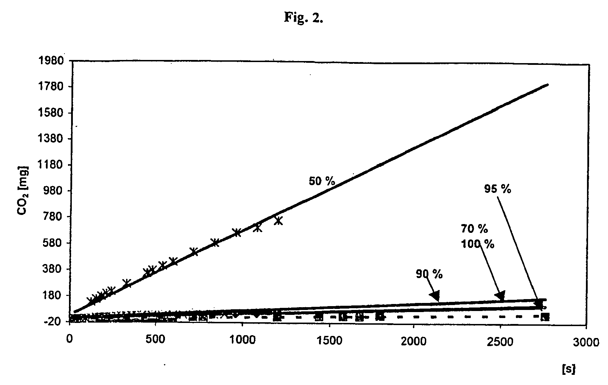

The influence of the percentage of oil on the emission rate is evident in Fig. 2. An optimum emission rate at an oil phase concentration of 50% (for this particular formulation) is observed. Other experiments carried out with diverse oil phases and hydrophilic polymers show a similar behavior (not shown).

**Example 4.** Influence of the molecular weight of the polymer on the emission rate of $CO_2$ of an alkaline tablet.

In the following example, benzoic acid incorporated in a tablet having silicone oil as oil phase, and propylene glycol 2000 or 5000 as polymer, as well as Aldo® as emulsifier, are used. The basic solution with which reaction is made is KOH 0.05 M.

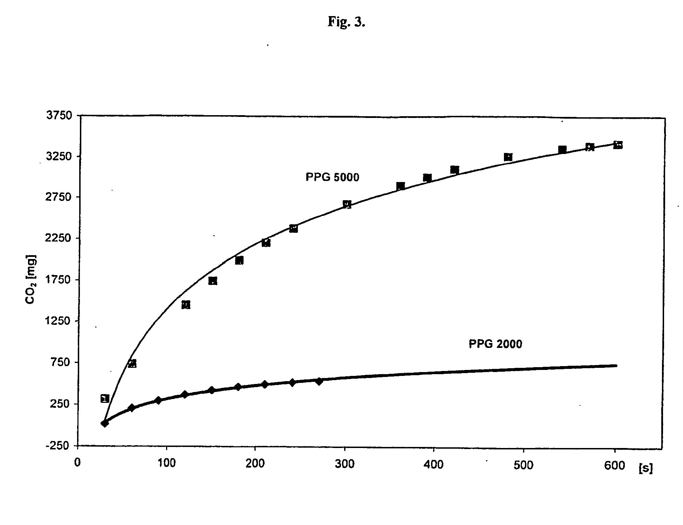

In Fig. 3 it is seen that upon the increase the molecular weight of propylene glycol (5000 amus), the emission is more appropriate. The inventors have confirmed that this behavior is not always the same, and in some instances, a polymer of smaller molecular weight has a more appropriate or greater emission rate of $CO_2$; the inventors have carried out satisfactory experiments with polymers ranging from 100 amus to 500000 amus (not shown), however, the best conditions are obtained in the range of 1000-30000 amus, and more preferably between 1000-7000 amus.

**Example 5.** Influence of the presence of salts on the emission of $CO_2$.

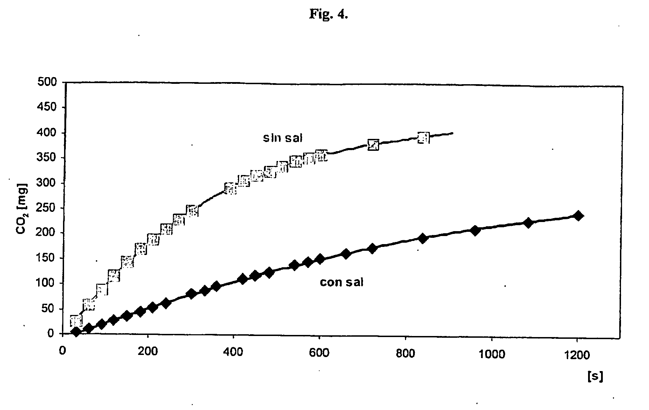

The influence of the presence -in the lactic acid solution (10% v/v) used to cause the reaction with a tablet containing barium carbonate- of potassium chloride (1 g / 500 mL of lactic acid) on the emission of $CO_2$ is seen in Fig. 4. The emulsifier that surrounds the barium carbonate particles acts as a semipermeable membrane through which water can pass but ions cannot move.

**Example 6.** Mixed tablets containing acid and base.

In a preferred embodiment mode of the invention, a granulated organic acid -oxalic acid- and a granulated organic base -barium carbonate- are added to the primary emulsion of the type described in the first example. The only risk in this process is that, before being covered by the emulsion or emulsified-dispersed, the base and acid begin to react since they are in contact (but in a solid state) during the process of mixing with the emulsion. For this, a perfectly appropriate measure is to work with anhydrous bases and alkalis and in a room with a dehumidifier for maintaining the humidity appropriately low (recommended below 30%).

The tablet made may be dissolved by a pure water solution (without other fundamental components) or even by solutions of alcohols at different concentrations in water.

A general process for the production of the desired sodium carbonate tablets, wherein several improvements and innovations with regards to state of the art, has been described:

1.- A constant emission rate of CO2 and without arbitrary emission of $CO_2$ peaks is achieved (see Figs. 1, 2, 3, and 4).

2.- For the process of manufacturing of $CO_2$ producing tablets, it is highly advisable that the alkaline compounds are anhydrous since the inventors have shown herein for the first time the importance of the humidity factor prior to manufacturing the tablets.

3.- The inventors have shown that when the alkali compound particles have a size greater than that desired (a diameter between 0,01 and 2 mm), the acid-base reaction takes place irregularly. A person skilled in the art could consider this circumstance foreseeable, however, only by means of the experiments carried out by the inventors is it possible to establish this point since we are talking about a complex emulsified matrix and either particle size could have shown to have little influence or it could have been convenient to use a particle size greater than that indicated. Obviously, larger-sized particles may be used within the scope of the invention but the range 0,01-0,5 mm has been preferably chosen.

4.- Unlike the majority of tablets described for $CO_2$ production (especially medicaments in effervescent formulae), the process of compacting the capsule is industrially simplified thanks to the compaction of the tablet due to the polymeric nature of the matrix, machinery then not being needed that exerts great pressure on the tablets for their appropriate compaction and longevity -industrially very expensive-.

5.- The tablets do not leave any toxic or persistent residues, unlike tablets for which compacting methods with very persistent polymers or with the presence of halogens are used.

6.- The inventors have developed an acid-base reaction retardant system (the following example talks about an alkaline tablet) by means of a completely novel technique. For a person skilled in the art it is obvious that by coating the alkali compound granules with an (vegetable or mineral) oil, the reaction with the acid is retarded by means of a controlled diffusion. Nevertheless, it is not obvious that that retardation is controlled, and for example, it could happen that the reaction is especially aggressive once an access of the acid to the base has been opened; or on the contrary, that the acid can never gain access to the alkaline compound. The innovation of the present invention lies in the use of an uncommon emulsion, made up of an oil phase and a hydrophilic phase -but not aqueous, as is usual-, so as to provide a barrier for the alkali granules, but with the special characteristic that the barrier is made up of a polymer allowing a retarded acid penetration, whereas the oil phase acts as a constant barrier during the whole process without gas explosions occurring within an oil phase (fonned $CO_2$ which destroys the tablet by pressure in an uncontrolled manner), provided this oil phase is in contact with a polymer that allows the passage of $CO_2$. The coating of active components, for their use in medicine, agricultural chemistry, etc., with polymers that degrade over time or with humidity is very common, however, the applicant has absolutely no knowledge of any publication or patent wherein the physical-chemical process is regulated by a polymer emulsion with solid material dispersed therein, in particular, for the regulation of the emission of gases.

7.- The inventors have developed other concepts applicable to this type of $CO_2$ producing pills, such as increasing the ionic strength of the solution in which the pill disintegrates, acting then in addition to the novel process described in the previous section, a control factor never described in combination with a structure of polymer emulsion with dispersed solids.

8.- The inventors describe the use of mixed, acid-base tablets that only react in the presence of water (not present in the state of the art) or in alcohol solutions, preferably of ethanol or isopropanol - because of their relative innocuousness-, with the special feature with regards to the prior state of the art that there is an emulsion that exerts great influence on reaction regularity.

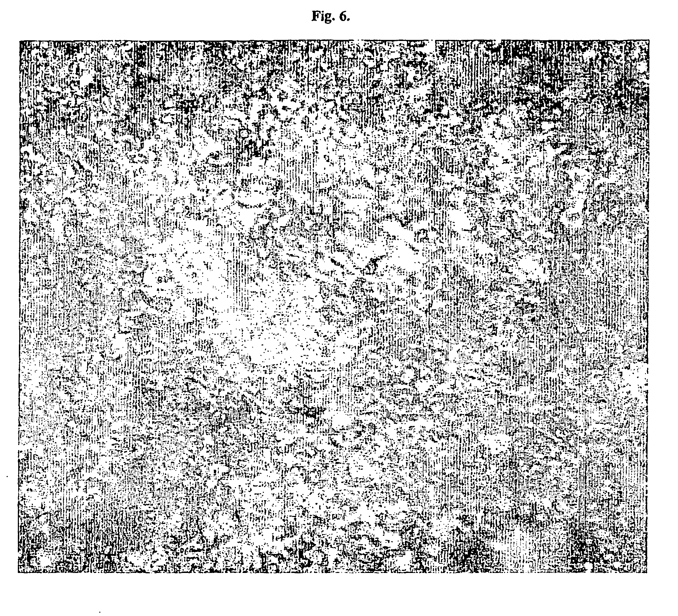

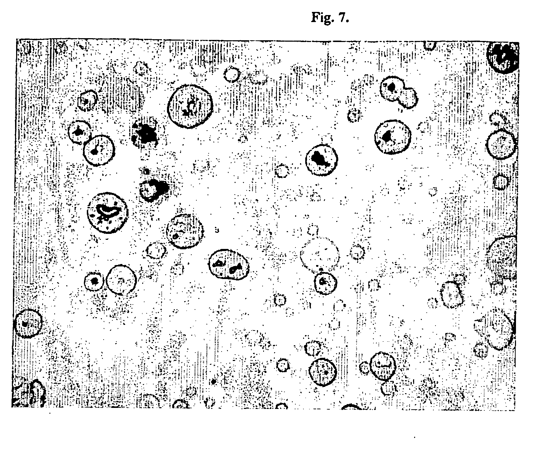

9.- The tablets described in the present invention, unlike almost the entirety of those existing in the state of the art, do not depend on, not even aim at, the use of the hydrophilic polymer for a gelification that improves the emission rate of the gas. This is evident from Figs. 6, 7, 8, and 9.

There are many variants within the object of the present patent. Some of them will be described in a non-limiting manner.

- The tablets may acquire a geometric shape such that the surface of contact with the acid solution remains constant for the longest possible time even though the tablet is shrinking (for example, by means of geometric shapes such as toruses, toruses with an inner and/or outer hyperbolic cosine surface, etc.), such that the "reaction contact surface" factor is eliminated from the reaction variables.

- The tablets may have several layers with diverse levels of polymer compaction or quantity in order either to have an emission for a very long time or to speed up or slow down the process in accordance with the $CO_2$ emission needs throughout the life of the tablet that is consumed. Specifically, a cylindrical tablet with three layers, with the top and bottom layers more firmly compacted than the intermediate layer, could be made. Three-layered tablets are described in FR 2784583, but there is no reaction-regulating emulsion in said patent, and in this invention, three layers are an example of a multitude of different formulations. The acid attack will be slower at the beginning, but if we are talking about a cylinder, the surface to be attacked is larger, and by means of strategies of this kind the emission rate can be controlled. In another embodiment, the tablets, for example sphere-shaped, could have a core with a compaction and/or greater presence of the oil phase for retarding the end of the tablet.

- The tablets may be made up of many components, organic or inorganic, such that they are capable of producing gases, as well as the tablets may be made up of alkaline or acid components which are solid at room or tablet usage temperature. In an embodiment of the invention, tartaric acid crystals are emulsified-dispersed in tablets in accordance with the procedure of Example 1.

- The primary emulsion may be both of oil in water and of water in oil, and the quantity of spaces filled with air in the tablet formulated by means of the agitation rate and mode when making the primary emulsion (incorporation of air into the emulsion), as well as at the final pressing made during the molding of the tablet, can be controlled.

- The tablets may be made up of mixtures of different bases, or alternatively, of different acids.

- Likewise, the tablets may contain at the same time physically separated acids and bases, and the gas producing reaction may be caused by a solution that dissolves-disintegrates the tablet and at the same time dissolves in a controlled manner the acids and bases in the tablet, the former then coming in contact and the gas generating acid-base reaction occurring next.

- The gases produced in each type of tablet-solution pair may be diverse, such as the production of $CO_2$ and $SH_2$ at the same time. In the last case, the tablets may be used for the purpose of causing a pungent smell appropriate for example in alarm devices (an alarm device causes the acid-base reaction to start, with the development of sulfur trioxide or dioxide, which works as an olfactory alarm, especially appropriate for deaf or blind people), in prank items, etc. A complete device for its use as alarm based on the described tablets is not an object of this patent, but the use of the described tablets and the acid-base reaction as olfactory alarm mechanism does fall within the object of the patent.

- The acids or bases used do not necessarily have to be solids, it being possible for them to be liquids at room temperature or solids at the tablet preparation temperature and liquids at room temperature.

- Likewise, the tablets may contain polymers that temporarily sequester the acid or alkali alternatively as a regulating measure of the reaction rate.

- Sequesterers may be also added to the tablets (e.g. EDTA) such that at first the acid or base (or mixture of acids or alkalis) is totally or partially sequestered, such that the reaction rate is thereby also regulated (this preferred embodiment of the invention is more suitable when we are talking about tablets of acids or alkalis liquid at room temperature, but not necessarily only applicable for this circumstance, since when the acid or the alkali is solid and is dissolved by the reacting solution, it can be temporarily sequestered or chelated, totally or partially, with regards to the total quantity of alkali or acid present.

- It is also possible to incorporate dissolved gases (e.g. methyl mercaptan) in the emulsion or the liquid acid or alkali for their subsequent emission when the tablet disintegrates.

- The oil phase may contain additives that prevent its microbial deterioration or rancidity.

- Both the polymer and the oil phase, as well as the emulsifier, as any additive, may be mixtures, such as for example a 1:1 mixture of polypropylene glycol and polyethylene glycol as polymer.

Many other variants of the present invention are possible and fall within the object of the invention insofar as the tablets are made up of a matrix based on an emulsion of oil and a hydrophilic phase of polymeric nature, which on contact with an appropriate solution, causes or is an essential part of an acid-base or enzymatic reaction generating one or various gases.

## Claims

1. Tablets for the controlled emission of at least one gas, **characterized in that** they comprise one emulsion-dispersion made up of:

    - an acid or an alkali or both, free or in the form of a salt or chelate, in a solid or liquid state dispersed in

- a water-in-oil or oil-in-water emulsion containing:

    (a) at least one emulsifier
    (b) at least one hydrophilic polymer
    (c) an oil phase
    (d) optionally, additives;

said tablets being solid at a temperature lower than 50 °C.

2. A process of production of a tablet for the controlled emission of at least one gas, **characterized in that** in a first step, an emulsion of an oil phase in a hydrophilic polymer is formed thanks to an appropriate emulsifier, under hot conditions and under agitation, and in a second step, an acid or an alkali or an alkali and acid mixture is alternatively added, which is mixed, under hot agitation, until a viscous liquid with an appropriate homogeneity level is obtained; the emulsion-dispersion thus formed is cooled, at the same time giving it the desired shape by means of molding; finally, the tablet is obtained in a state solid and stable against unintentional disintegration.

3. Tablets for the controlled emission of at least one gas according to the preceding claim, **characterized in that** the solid state is basically achieved by temperature drop after hot forming the paste essentially constituting the tablet and not by pressure, a certain light pressure being necessary only for molding.

4. A tablet consisting of an emulsion-dispersion for the controlled emission of gas according to any appropriate combination of the preceding claims, **characterized in that** the hydrophilic polymer has a molecular weight of 100-500000 amus, preferably of 1000-30000 amus, and more preferably between 1000-8000 amus.

5. A tablet consisting of an emulsion-dispersion for the controlled emission of gas according to any appropriate combination of the preceding claims, **characterized in that** the oil phase comprises compounds chosen among the group of: natural or modified vegetable oils, hydrogenated vegetable oils, mineral oils, silicones, fluorinated silicones, modified silicones, or mixtures thereof in any proportion.

6. A tablet consisting of an emulsion-dispersion for the controlled emission of gas according to any appropriate combination of the preceding claims, **characterized in that** the oil phase or the hydrophilic polymer or both contain additives:

    (a) that prevent the deterioration thereof, preferably antimicrobial agents, antioxidants, UV blockers or filters.
(b) odorizing aromas that give off a characteristic smell, together with the evolution of gas, upon said tablets reacting with an acid or a base.
(c) sequesterers of acids or bases.
(d) adsorbents/sequesterers of acids or bases of an organic, preferably EDTA, protein complexes, or inorganic, e.g, clays, sepiolites, zeolites, nature, including polymers, preferably based on citric or maleic acid remains integrated in the polymer, in the case of an alkaline tablet, preferably based on sulphonates, lignosulphonates, in the case of an acid tablet,
(e) salts that increase the ionic strength of the solution in which the tablet disintegrates.
(f) agglomerants or antiagglomerants, dispersants, stabilizers.

7. A tablet consisting of an water-in-oil emulsion-dispersion for the controlled emission of gas according to any appropriate combination of the preceding claims, **characterized in that** the emulsifier or mixture of emulsifiers has a hydrophilic-lipophilic balance HLB of 1-7.

8. A tablet consisting of an oil-in-water emulsion-dispersion for the controlled emission of gas according to any appropriate combination of the preceding claims, **characterized in that** the emulsifier or mixture of emulsifiers has a hydrophilic-lipophilic balance HLB of 7-18.

9. A tablet consisting of an emulsion-dispersion for the controlled emission of gas according to any appropriate combination of the preceding claims, **characterized in that** the hydrophilic phase is chosen among the group of polymers of the polyalkylene glycols, optionally derivatized.

10. A tablet according to any appropriate combination of the preceding claims, **characterized in that** the generated gas is at least $CO_2$.

11. A tablet according to claim 10, **characterized in that** the gas comes from a carbonate or a hydrogen carbonate of an alkaline or alkaline-earth metal or its mixtures emulsified-dispersed inside said tablet, the $CO_2$ gas being released by reaction of any acid in the presence of the tablet.

12. A tablet according to any appropriate combination of the preceding claims, **characterized in that** the acids are preferably chosen among the group: citric, lactic, phosphoric, benzoic, malic, maleic, malonic, fumaric, acetic, formic, propionic, succinic; as well as salts from these acids; these acids or their mixtures being emulsified-dispersed inside the tablet.

13. A tablet according any appropriate combination of the preceding claims, **characterized in that** it generates $CO_2$ gas and **in that** the preferred acids mentioned in Claim 12 are in a solution that reacts with an alkaline tablet.

14. A tablet according any appropriate combination of the preceding claims, **characterized in that** it generates $CO_2$ gas and **in that** the preferred bases mentioned in Claim 11 are found in a solution that reacts with an acid tablet.

15. A tablet according to any appropriate combination of the preceding claims, **characterized in that** it generates $CO_2$ gas and **in that** both the acid(s) and the alkali(s) are found emulsified-dispersed in the same tablet, the mixture and thus the gas releasing reaction occurring upon the disintegration of the tablet in a medium that allows the reaction between the acid(s) and the alkali(s).

16. The use of tablets according to any appropriate combination of the preceding claims, **characterized in that** they are incorporated in a drip-type medical device wherein there is an aqueous solution of an acid, preferably citric acid at 30-37% in water (w/w), separated by means of a sheet of plastic or plastic with aluminum from an alkaline tablet, preferably consisting of (a) partially hydrogenated vegetable oil (b) emulsified with a hydrophilic polymer, most preferably of a molecular mass between 2000-8000 amus, (c) an emulsifier of HLB 3-6, and (d) an alkali, preferably sodium carbonate; the sheet being broken by pressure right at the desired moment of using the drip; the $CO_2$ gas is then released by a controlled acid-base reaction upon the citric acid coming in contact with the bicarbonate and carbonate ions, and enters a chamber wherein the injectable liquid solution, which is encased in plastic, is found, preferably saline serum, serums with medicaments, blood or products derived from blood encased in plastic, the $CO_2$ thereby creating pressure on the casing of the injectable solution which generates its flow towards the patient, this pressure being the primary regulator of the rate of injection of the injectable solution.

17. The use of tablets according to any appropriate combination of the preceding claims, **characterized in that** a controlled emission of pungent smell by the release of a pungent gas such as sulfur or ammonia or mercaptan compounds -or their mixtures-, more preferably $SH_2$ is obtained in an olfactory alarm device, wherein the factor causing the alarm state trip causes in turn that an acid or basic tablet contacts a basic or acid solution respectively.

18. The use of tablets according to any appropriate combination of the preceding claims in agriculture for the disinfection of soils or cultivation sites in general, by the release of disinfecting and/or fumigating gases traditionally used or present in the state of the art, preferably hydrogen cyanide.

19. Tablets according to any appropriate combination of the preceding claims, **characterized in that** they contain an emetic product as an additive so that they are vomited in case of voluntary or involuntary ingestion thereof, greatly reducing their toxicity due to ingestion.

20. The use of tablets according to any appropriate combination of the preceding claims, **characterized in that** said tablets act as aromatic agents in the bath/shower upon being dissolved in water or in home aroma devices, by disintegration thereof in contact with water at the usual pH of the bath or home water; the aromas preferably being chosen among the group of: thymol, borneol, anethole, limonene, pinene, and terpenes in general.

21. Tablets for the controlled emission of $O_2$, **characterized in that** they comprise a water-in-oil emulsion made up of:

    (a) at least one emulsifier
    (b) at least one hydrophilic polymer
    (d) hydrogen peroxide
    (d) an oil phase
    (e) optionally, additives;

    said tablets being molded in a solid state at a temperature lower than 40 °C and applicable in controlled breathing systems both at a microbial and an animal or plant level, as well as in systems or processes requiring a continuous $O_2$ supply in a controlled manner, preferably chemical reactions, aquariums and chambers for microorganism growing.

22. Tablets according to any appropriate combination of the preceding claims, **characterized in that** they generate $O_2$ gas and **in that** the tablets formed according to Claim 21 come in contact with a solution containing enzymes of the peroxidase group, this solution produces $O_2$ upon the enzymes coming in contact with the hydrogen peroxide which is found in the tablet as a dispersed phase, together with the hydrophilic polymer, the oil being the continuous phase.

23. Tablets according to any possible combination of the preceding claims, wherein a colorant or reaction indicator (either acid-base or enzymatic) is added in order to observe the appearance of color upon initiation of the reaction, optionally the intensity of the color varies as the reaction develops.

**Patentansprüche**

1. Tabletten für die kontrollierte Emission von mindestens einem Gas, **dadurch gekennzeichnet, dass** sie eine Emulsion/Dispersion aufweisen, die aus Folgendem besteht:

- einer Säure oder einer Lauge oder aus beidem, die frei oder in der Form eines Salzes oder Chelats in einem festen oder flüssigen Zustand vorliegen und in

- einer Wasser-in-Öl- oder Öl-in-Wasser-Emulsion verteilt sind, die Folgendes enthält:

(a) mindestens einen Emulgator
(b) mindestens ein hydrophiles Polymer
(c) eine Ölphase
(d) Zusatzstoffe nach Wahl;

wobei die Tabletten bei einer Temperatur unter 50°C fest sind.

2. Verfahren der Herstellung einer Tablette für die kontrollierte Emission von mindestens einem Gas, **dadurch gekennzeichnet, dass** in einem ersten Schritt dank eines geeigneten Emulgators unter heißen Bedingungen und unter Bewegung eine Emulsion aus einer Ölphase in einem hydrophilen Polymer gebildet wird und in einem zweiten Schritt eine Säure oder eine Lauge oder alternativ ein Gemisch aus einer Säure und einer Lauge zugegeben wird; welche bei Bewegung unter heißen Bedingungen vermischt werden, bis eine visköse Flüssigkeit mit einem geeigneten Homogenitätsgrad erhalten wird, die so gebildete Emulsion-Dispersion gleichzeitig gekühlt wird, wobei sie gleichzeitig durch Abformung die gewünschte Form erhält, und die Tablette schließlich in einem festen und gegen unbeabsichtigten Zerfall stabilen Zustand erhalten wird.

3. Tabletten für die kontrollierte Emission von mindestens einem Gas nach dem vorherigen Anspruch, **dadurch gekennzeichnet, dass** der feste Zustand im Wesentlichen durch einen Temperaturabfall nach dem Heißformen der Paste, aus welcher die Tablette im Wesentlichen gebildet ist, und nicht durch Druck erreicht wird, wobei lediglich für die Abformung ein gewisser leichter Druck erforderlich ist.

4. Aus einer Emulsion-Dispersion bestehende Tablette für die kontrollierte Emission von Gas nach einer geeigneten Kombination der vorherigen Ansprüche, **dadurch gekennzeichnet, dass** das hydrophile Polymer ein Molekulargewicht von 100-500000 amu, vorzugsweise von 1000-30000 amu und mehr bevorzugt zwischen 1000 und 8000 amu aufweist.

5. Aus einer Emulsion-Dispersion bestehende Tablette für die kontrollierte Emission von Gas nach einer geeigneten Kombination der vorherigen Ansprüche, **dadurch gekennzeichnet, dass** die Ölphase Verbindungen in einem beliebigen Anteil aufweist, die aus der Gruppe natürlicher oder modifizierter pflanzlicher Öle, hydrogenierten pflanzlichen Ölen, Mineralölen, Silikonen, fluorierten Silikonen oder deren Gemischen ausgewählt sind.

6. Aus einer Emulsion-Dispersion bestehende Tablette für die kontrollierte Emission von Gas nach einer geeigneten Kombination der vorherigen Ansprüche, **dadurch gekennzeichnet, dass** die Ölphase oder das hydrophile Polymer oder beide folgende Zusatzstoffe enthalten:

(a) Zusatzstoffe, welche ihre Zerstörung verhindern, vorzugsweise antimikrobielle Stoffe, Antioxidanzien, UV-Blocker oder -Filter;
(b) Geruch spendende Aromastoffe, welche zusammen mit der Bildung von Gas, wenn die Tabletten mit einer Säure oder einem Gas reagieren, einen charakteristischen Geruch abgeben;
(c) Bindemittel von Säuren oder Basen;
(d) Adsorptionsmittel/Bindemittel von Säuren oder Basen organischer Art, vorzugsweise EDTA, Proteinkomplexe, oder anorganischer Art wie z. B. Tone, Sepiolite, Zeolite, einschließlich von Polymeren, bei einer alkalischen Tablette vorzugsweise basierend auf in das Polymer integrierten Zitronen- oder Maleinsäurerückständen und bei einer sauren Tablette vorzugsweise basierend auf Sulfonaten, Lignosulfonaten.
(e) Salze, welche die Ionenstärke der Lösung, in der die Tablette zerfällt, erhöht;
(f) Agglomerationsmittel bzw. Antiagglomerationsmittel, Dispersionsmittel, Stabilisatoren.

7. Aus einer Wasser-in-Öl-Emulsion-Dispersion bestehende Tablette für die kontrollierte Emission von Gas nach einer geeigneten Kombination der vorherigen Ansprüche, **dadurch gekennzeichnet, dass** der Emulgator oder das Emulgatorgemisch ein hydrophiles-lipophiles Gleichgewicht Hydrophilic-Lipophilic Balance, HLB von 1 bis 7 aufweist,

8. Aus einer Öl-in-Wasser-Emulsion-Dispersion bestehende Tablette für die kontrollierte Emission von Gas nach einer geeigneten Kombination der vorherigen Ansprüche, **dadurch gekennzeichnet, dass** der Emulgator oder das Emulgatorgemisch ein hydrophiles-lipophiles Gleichgewicht Hydrophilic-Lipophilic Balance, HLB von 7 bis 18 aufweist.

9. Aus einer Emulsion-Dispersion bestehende Tablette für die kontrollierte Emission von Gas nach einer geeigneten Kombination der vorherigen Ansprüche, **dadurch gekennzeichnet, dass** die hydrophile Phase aus der Polymergruppe der Polyalkylenglycole, wahlweise derivatisiert, ausgewählt wird.

10. Tablette nach einer geeigneten Kombination der vorherigen Ansprüche, **dadurch gekennzeichnet, dass** es sich bei dem erzeugten Gas um mindestens $CO_2$ handelt.

11. Tablette nach Anspruch 10, **dadurch gekennzeichnet, dass** das Gas von einem Carbonat- oder einem Hydrogencarbonat eines Alkali- oder Alkalierdmetalls oder seinen Gemischen stammt, das in der Tablette emulgiert-dispergiert ist, wobei das $CO_2$-Gas durch eine Säurereaktion in Gegenwart der Tablette freigesetzt wird.

12. Tablette nach einer geeigneten Kombination der vorherigen Ansprüche, **dadurch gekennzeichnet, dass** die Säuren vorzugsweise aus der Gruppe Zitronen-, Milch-, Phosphor-, Benzoe-, Äpfel-, Malein-, Malon-, Fumar-, Essig-, Ameisen-, Propion-, Bernsteinsäure sowie aus Salzen dieser Säuren ausgewählt sind, wobei diese Säuren oder ihre Gemische in der Tablette emulgiert-dispergiert sind.

13. Tablette nach einer geeigneten Kombination der vorherigen Ansprüche, **dadurch gekennzeichnet, dass** sie $CO_2$-Gas erzeugt und dass die in Anspruch 12 erwähnten bevorzugten Säuren in einer Lösung vorliegen, die mit einer alkalischen Tablette reagiert.

14. Tablette nach einer geeigneten Kombination der vorherigen Ansprüche, **dadurch gekennzeichnet, dass** sie $CO_2$-Gas erzeugt und dass die in Anspruch 11 erwähnten bevorzugten Basen in einer Lösung vorkommen, die mit einer sauren Tablette reagiert.

15. Tablette nach einer geeigneten Kombination der vorherigen Ansprüche, **dadurch gekennzeichnet, dass** sie $CO_2$-Gas erzeugt und dass sowohl die Säure(n) als auch die Lauge(n) emulgiert-dispergiert in derselben Tablette vorkommt bzw. vorkommen, wobei die Mischung und daher die Gas freisetzende Reaktion bei dem Zerfall der Tablette in einem Medium stattfinden, welches die Reaktion zwischen der Säure bzw. den Säuren und der Lauge bzw. den Laugen gestattet.

16. Verwendung von Tabletten nach einer geeigneten Kombination der vorherigen Ansprüche, **dadurch gekennzeichnet, dass** sie in eine tropfartige medizinische Vorrichtung integriert sind, in der sich eine wässrige Lösung einer Säure befindet, vorzugsweise 30-37% Zitronensäure in Wasser (Gew./Gew.) und durch eine Folie aus Kunststoff oder Kunststoff mit Aluminium von einer alkalischen Tablette getrennt sind, vorzugsweise bestehend aus (a) teilweise hydrogeniertem pflanzlichem Öl, (b) das mit einem hydrophilen Polymer emulgiert ist, am meisten bevorzugt mit einer molekularen Masse zwischen 2000 und 8000 amu, (c) einem Emulgator von HLB 3-6 und (d) einer Base, vorzugsweise Natriumcarbonat, wobei die Folie genau im gewünschten Moment der Anwendung des Tropfs zerbrochen wird, das $CO_2$-Gas anschließend durch eine kontrollierte Säure-Base-Reaktion freigesetzt wird, indem die Zitronensäure mit den Bicarbonat- und Carbonationen in Berührung kommt, und in eine Kammer gelangt, in der sich die injizierbare flüssige Lösung, vorzugsweise Kochsalzserum, Sera mit Medikamenten, Blut oder aus Blut gewonnenen Produkten, in einem Kunststoffgehäuse befindet, wodurch das $CO_2$ einen Druck auf das Gehäuse der injizierbaren Lösung erzeugt, was deren Fluss zum Patienten hin erzeugt, wobei dieser Druck der primäre Regulator der Geschwindigkeit der Injektion der injizierbaren Lösung ist.

17. Verwendung von Tabletten nach einer geeigneten Kombination der vorherigen Ansprüche, **dadurch gekennzeichnet, dass** durch die Freisetzung eines stechenden Gases wie beispielsweise Schwefel oder Ammoniak oder Mercaptanverbindungen - oder deren Gemische -, mehr bevorzugt $SH_2$, in einer olfaktorischen Alarmvorrichtung eine kontrollierte Emission eines stechenden Geruchs erhalten wird, wobei der Faktor, welcher den Alarmzustand auslöst, wiederum bewirkt, dass eine saure oder basische Tablette in Berührung mit einer basischen bzw. sauren Lösung gelangt.

18. Verwendung von Tabletten nach einer geeigneten Kombination der vorherigen Ansprüche in der Landwirtschaft für die Desinfektion von Böden oder Anbaustellen im Allgemeinen durch die Freisetzung von desinfizierenden Gasen, die traditionell verwendet werden oder im Stand der Technik vorhanden sind, vorzugsweise Wasserstoffcyanid.

19. Tabletten nach einer geeigneten Kombination der vorherigen Ansprüche, **dadurch gekennzeichnet, dass** sie ein emetisches Produkt als Zusatzstoff enthalten, so dass sie im Fall ihres freiwilligen oder unfreiwilligen Verschluckens erbrochen werden, was ihre verdauungsbedingte Toxizität in hohem Maß verringert.

20. Verwendung von Tabletten nach einer geeigneten Kombination der vorherigen Ansprüche, **dadurch gekennzeichnet, dass** die Tabletten, indem sie bei Berührung mit Wasser des üblichen pH-Wertes des Badewassers oder des häuslichen Wassers zerfallen, als Aromastoffe im Bad/in der Dusche wirken, wenn sie in Wasser gelöst werden, oder in häuslichen Duftvorrichtungen als Aromastoffe wirken, wobei die Aromen vorzugsweise aus der Gruppe Thymol, Bomeol, Anethol, Limonen, Pinen und Terpenen im Allgemeinen ausgewählt sind.

21. Tabletten für die kontrollierte Emission von $CO_2$, **dadurch gekennzeichnet, dass** sie eine Wasser-in-Öl-Emulsion aufweisen, die Folgendes enthält:

    (a) mindestens einen Emulgator
    (b) mindestens ein hydrophiles Polymer
    (c) Wasserstoffperoxid
    (d) eine Ölphase
    (e) Zusatzstoffe nach Wahl;

wobei die Tabletten in einem festen Zustand bei einer Temperatur unter 40°C geformt werden und in Systemen kontrollierter Atmung sowohl auf mikrobieller als auch auf tierischer oder pflanzlicher Ebene sowie in Systemen oder Verfahren anwendbar sind, die eine kontinuierliche und kontrollierte $O_2$-Zufuhr benötigen, vorzugsweise chemische Reaktionen, Aquarien und Kammern für die Anzucht von Mikroorganismen.

22. Tabletten nach einer geeigneten Kombination der vorherigen Ansprüche, **dadurch gekennzeichnet, dass** sie $O_2$-Gas erzeugen und dass die nach Anspruch 21 geformten Tabletten mit einer Lösung in Berührung kommen, welche Enzyme aus der Peroxidasegruppe enthält, diese Lösung $O_2$ produziert, wenn die Enzyme mit dem Wasserstoffperoxid in Berührung kommen, das zusammen mit dem hydrophilen Polymer in der Tablette als dispergierte Phase vorliegt, wobei das Öl die kontinuierliche Phase ist,

23. Tabletten nach einer geeigneten Kombination der vorherigen Ansprüche, wobei ein Farbstoff oder ein Reaktionsindikator (entweder Säure-Base oder enzymatisch) zugegeben wird, um das Erscheinen einer Farbe nach Ingangsetzen der Reaktion zu beobachten, wobei sich wahlweise die Intensität der Farbe ändert, während sich die Reaktion entwickelt.

## Revendications

1. Comprimés pour l'émission contrôlée d'au moins un gaz, **caractérisés en ce qu'**ils comprennent une émulsion-dispersion constituée de :

    - un acide ou un alcali ou les deux, libre ou sous forme d'un sel ou un chélate, à l'état solide ou liquide dispersé dans :
    - une émulsion eau dans l'huile ou huile dans l'eau contenant :

        (a) au moins un agent émulsifiant
        (b) au moins un polymère hydrophile

        (c) une phase huile
        (d) optionnellement, des additifs ;

lesdits comprimés étant solides à une température inférieure à 50°C.

2. Procédé de production d'un comprimé pour l'émission contrôlée d'au moins un gaz, **caractérisé en ce que** dans une première étape, une émulsion d'une phase huile est formée dans un polymère hydrophile grâce à un agent émulsifiant approprié, dans des conditions chaudes et sous agitation, et dans une deuxième étape, on ajoute alternativement un acide ou un alcali ou un mélange d'alcali et acide, lequel est mélangé, sous agitation chaude, jusqu'à l'obtention d'un liquide visqueux ayant un niveau approprié d'homogénéité ; on refroidit l'émulsion dispersion ainsi formée tout en lui donnant la forme voulue au moyen de moulage ; finalement, on obtient le comprimé à l'état solide et stable par rapport à la désintégration involontaire.

3. Comprimés pour l'émission contrôlée d'au moins un gaz selon la revendication précédente, **caractérisés en ce que** l'on atteint essentiellement l'état solide par une chute de température après le formage à chaud de la pâte constituant essentiellement le comprimé et non pas par pression, une certaine légère pression étant nécessaire seulement au moulage.

4. Comprimé consistant en une émulsion-dispersion pour l'émission contrôlée de gaz selon une combinaison appropriée quelconque des revendications précédentes, **caractérisé en ce que** le polymère hydrophile a un poids moléculaire de 100-500000 u, de préférence de 1000-30000 u, et plus de préférence ente 1000-8000 u.

5. Comprimé consistant en une émulsion-dispersion pour l'émission contrôlée de gaz selon une combinaison appropriée quelconque des revendications précédentes, **caractérisé en ce que** la phase huile comprend des composés choisis parmi le groupe des : huiles végétales naturelles ou modifiées, huiles végétales hydrogénées, huiles minérales, silicones, silicones fluorés, silicones modifiés ou leurs mélanges dans une proportion quelconque

6. Comprimé consistant en une émulsion-dispersion pour l'émission contrôlée de gaz selon une combinaison appropriée quelconque des revendications précédentes, **caractérisé en ce que** la phase huile ou le polymère hydrophile ou les deux comprennent des additifs :

    (a) qui préviennent leur détérioration, de préférence des agents antimicrobiens, des antioxydants, des bloqueurs ou filtres UV.

(b) des arômes odorants qui dégagent une odeur caractéristique, en même temps que l'évolution de gaz, lors de la réaction desdits comprimés avec un acide ou une base.

(c) des séquestrants d'acides ou de bases.

(d) des adsorbants/séquestrants d'acides ou de bases de nature organique, de préférence EDTA, complexes de protéines, ou de nature inorganique, par exemple, des argiles, sépiolites et zéolites, incluant des polymères, de préférence basés sur des restes d'acide citrique ou maléique intégrés dans le polymère, dans le cas d'un comprimé alcalin, de préférence basés sur des sulfonates, des lignosulfonates, dans le cas d'un comprimé acide.

(e) des sels qui augmentent la force ionique de la solution dans laquelle le comprimé se désintègre.

(f) des agglomérants ou des antiagglomérants, des dispersants, des stabilisants.

7. Comprimé consistant en une émulsion-dispersion d'eau dans l'huile pour l'émission contrôlée de gaz selon une combinaison appropriée quelconque des revendications précédentes, **caractérisé en ce que** l'agent émulsifiant ou le mélange d'agents émulsifiants a un bilan hydrophile-lipophile BHL allant de 1 à 7.

8. Comprimé consistant en une émulsion-dispersion d'huile dans l'eau pour l'émission contrôlée de gaz selon une combinaison appropriée quelconque des revendications précédentes, **caractérisé en ce que** l'agent émulsifiant ou le mélange d'agents émulsifiants a un bilan hydrophile-lipophile BHL allant de 7 à 18.

9. Comprimé consistant en une émulsion-dispersion pour l'émission contrôlée de gaz selon une combinaison appropriée quelconque des revendications précédentes, **caractérisé en ce que** l'on choisit la phase hydrophile parmi le groupe de polymères des glycols de polyalkylène, optionnellement dérivés.

10. Comprimé selon une combinaison appropriée quelconque des revendications précédentes, **caractérisé en ce que** le gaz généré est au moins du $CO_2$.

11. Comprimé selon la revendication 10, **caractérisé en ce que** le gaz provient d'un carbonate ou un carbonate d'hydrogène d'un métal alcalin ou alcalino-terreux ou ses mélanges émulsifié-dispersé au sein dudit comprimé, le gaz $CO_2$ étant libéré par réaction d'un acide quelconque en présence du comprimé.

12. Comprimé selon une combinaison appropriée quelconque des revendications précédentes, **caractérisé en ce que** l'on choisit les acides de préférence parmi le groupe des acides : citrique, lactique, phosphorique, benzoïque, malique, maléique, malonique, fumarique, acétique, formique, propionique, succinique ; ainsi que des sels provenant de ces acides ; ces acides ou leurs mélanges étant émulsifiés-dispersés au sein du comprimé.

13. Comprimé selon une combinaison appropriée quelconque des revendications précédentes, **caractérisé en ce qu'**il génère du gaz $CO_2$ et **en ce que** les acides préférés précités dans la revendication 12 sont dans une solution qui réagit avec un comprimé alcalin.

14. Comprimé selon une combinaison appropriée quelconque des revendications précédentes, **caractérisé en ce qu'**il génère du gaz $CO_2$ et **en ce que** les bases préférées précitées dans la revendication 11 se trouvent dans une solution qui réagit avec un comprimé acide.

15. Comprimé selon une combinaison appropriée quelconque des revendications précédentes, **caractérisé en ce qu'**il génère du gaz $CO_2$ et **en ce qu'**aussi bien le(s) acide(s) que les alcali(s) se trouvent émulsifiés-dispersés dans le même comprimé, le mélange et de la sorte la réaction de libération de gaz ayant lieu lors de la désintégration du comprimé dans un milieu qui permet la réaction entre le(s) acide(s) et le(s) alcali(s).

16. Utilisation de comprimés selon une combinaison appropriée quelconque des revendications précédentes, **caractérisé en ce qu'**ils sont incorporés dans un dispositif médical du type goutte-à-goutte dans lequel il y a une solution aqueuse d'un acide, de préférence de l'acide citrique dans une proportion de 30-37% en eau (p/p), séparée au moyen d'une feuille en plastique ou en plastique avec de l'aluminium d'un comprimé alcalin, consistant de préférence en (a) de l'huile végétale partiellement hydrogénée, (b) émulsifiée avec un polymère hydrophile, plus de préférence en une masse moléculaire entre 2000 et 8000 u, (c) un émulsifiant ayant un BHL allant de 3 à 6, et (d) un alcali, de préférence du carbonate de sodium ; la feuille étant cassée par la pression au moment précis et voulu de l'utilisation du goutte-à-goutte ; le gaz $CO_2$ est alors libéré par une réaction acide-base contrôlée lorsque l'acide citrique entre en contact avec les ions de bicarbonate et de carbonate, et pénètre dans une chambre dans laquelle se trouve la solution liquide injectable, qui est enveloppé dans du plastique, de préférence du sérum salin, des sérums avec des médicaments, du sang ou des produits dérivés du sang enveloppé dans du plastique, le $CO_2$ créant ainsi de la pression sur l'enveloppe de la solution injectable qui génère son écoulement vers le patient, cette pression étant le régulateur primaire de la vitesse d'injection de la solution injectable.

17. Utilisation de comprimés selon une combinaison appropriée quelconque des revendications précédentes, **caractérisé en ce que** l'on obtient une émission contrôlée d'une odeur âcre par la libération d'un gaz âcre tel que le soufre ou l'ammoniac ou des composés de mercaptan -ou leurs mélanges-, de préférence du $SH_2$, dans un dispositif d'alarme olfactive, dans lequel le facteur causant le déclenchement d'état d'alarme fait à son tour qu'un comprimé acide ou basique entre en contact respectivement avec une solution basique ou acide.

18. Utilisation de comprimés selon une combinaison appropriée quelconque des revendications précédentes dans l'agriculture pour la désinfection des sols ou des sites de culture en général, par la libération de gaz de désinfection et/ou de fumigation traditionnellement utilisés ou étant présents dans l'état de l'art, de préférence du cyanure d'hydrogène.

19. Comprimés selon une combinaison appropriée quelconque des revendications précédentes, **caractérisés en ce qu'**ils contiennent un produit émétique en tant qu'additif de manière à ce qu'ils soient vomis en cas d'ingestion volontaire ou involontaire de ceuxci, en réduisant en grande mesure leur toxicité due à l'ingestion.

20. Utilisation de comprimés selon une combinaison appropriée quelconque des revendications précédentes, **caractérisé en ce que** lesdits comprimés agissent comme des agents aromatiques dans le bain/la douche en se dissolvant dans l'eau ou dans les dispositifs d'arôme à utilisation domestique, par leur désintégration en contact avec l'eau ayant le pH usuel de l'eau de bain ou de l'eau domestique ; les arômes étant de préférence choisis parmi le groupe de : thymol, boméol, anéthol, limonène, pinène et terpènes en général.

21. Comprimés pour l'émission contrôlée de $CO_2$, **caractérisés en ce qu'**ils comprennent une émulsion eau dans l'huile constituée de :

(a) au moins un agent émulsifiant
(b) au moins un polymère hydrophile
(c) du peroxyde d'hydrogène
(d) une phase huile
(e) optionnellement, des additifs ;

lesdits comprimés étant moulés à l'état solide à une température inférieure à 40°C et applicables dans des systèmes respiratoires contrôlés aussi bien à un niveau microbien qu'animal ou végétal, ainsi que dans des systèmes ou des procédés nécessitant une alimentation continue en $O_2$ d'une manière contrôlée, de préférence des réactions chimiques, des aquariums et des chambres pour la culture de microorganismes.

22. Comprimés selon une combinaison appropriée quelconque des revendications précédentes, **caractérisés en ce qu'**ils génèrent du gaz $O_2$ et **en ce que** les comprimés formés selon la revendication 21 entrent en contact avec une solution contenant des enzymes du groupe de peroxydases, cette solution produit du $O_2$ lorsque les enzymes entre en contact avec le peroxyde d'hydrogène qui se trouve dans le comprimé sous forme de phase dispersée, en même temps que le polymère hydrophile, l'huile étant la phase continue.

23. Comprimés selon une combinaison appropriée quelconque des revendications précédentes, dans lesquels on ajoute un colorant ou un indicateur de réaction (soit acide-base ou enzymatique) afin d'observer l'aspect de la couleur lors du commencement de la réaction, optionnellement l'intensité de la couleur varie au fur et à mesure que la réaction se développe.

Fig. 1.

Fig. 2.

Fig. 3.

**Fig. 4.**

Fig. 5.

Fig. 6.

Fig. 7.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 27845832
- EP 0669129 A
- FR 2762213
- WO 9811879 A
- FR 2784583